# EUROPÄISCHE PATENTSCHRIFT

(11) **EP 0 528 128 B1**
(45) Veröffentlichungstag und Bekanntmachung des Hinweises auf die Patenterteilung: **20.11.1996**
(21) Anmeldenummer: 92109635.0
(22) Anmeldetag: 09.06.1992
(51) Int. Cl.: A61B 17/56

(54) **Verriegelungsnagel zur Versorgung von Femurfrakturen im mittleren und im trochanteren Bereich**
Centromedullary nail for treatment of central and trochanteral fractives of the femur
Clou centromédullaire destiné ou traitement de fractures centrales et trochantértiennes du fémur

(30) Priorität: 09.08.1991 DE 9109883 U
(43) Veröffentlichungstag der Anmeldung: 24.02.1993
(73) Patentinhaber: Howmedica GmbH, D-24232 Schönkirchen (DE)
(72) Erfinder: Grosse, Arsène Jérome, Dr., F-67000 Strassburg (FR); Harder, Hans Erich, W-2316 Probsteierhagen (DE)
(74) Vertreter: Patentanwälte Hauck, Graalfs, Wehnert, Döring, Siemons

(56) Entgegenhaltungen:
- EP-A- 0 192 840
- EP-A- 0 257 118
- EP-A- 0 306 709
- EP-A- 0 355 411
- DE-B- 1 248 228
- GB-A- 2 209 947
- US-A- 3 433 220

## Beschreibung

Die Erfindung bezieht sich auf einen Verriegelungsnagel zur Versorgung von Femurfrakturen im mittleren und im trochanteren Bereich nach dem Oberbegriff des Anspruchs 1.

Aus der EP-PS 0 257 118 ist ein Osteosynthesehilfsmittel zur Versorgung subtrochanterer Frakturen bekannt, das einen geschlossenen Verriegelungsnagel aufweist, der in seinem proximalen Bereich eine Schrägdurchbohrung zur Führung und Halterung einer Schenkelhalsschraube hat. Da der auch als Gamma-Nagel bekannte Verriegelungsnagel nicht selbst zur Frakturversorgung des Femurschafts dient, ist er kürzer als üblich ausgebildet und weist in seinem distalen Bereich lediglich Querbohrungen auf, um den Nagel axial und in Drehrichtung festzulegen. Die Schenkelhalsschraube sitzt passend in der schrägen Durchbohrung und ist daher gegen ein Verschwenken in der von ihr aufgespannten Ebene und um eine durch den Verriegelungsnagel gehende Achse wirksam gesichert. Zur Sicherung in Drehrichtung der Schenkelhalsschraube ist eine Drehsicherung, z.B. ein Verriegelungsstift, vorgesehen. Diese Drehsicherung läßt eine axiale Bewegung der Schenkelhalsschraube zu, wodurch die Schraube einer während der Knochenheilung auftretenden Knochenverkürzung nachgeben kann.

Während der aus der EP-PS 0 257 118 bekannte Verriegelungsnagel ein geschlossenes Profil aufweist, kann der sogenannte Küntscher-Nagel ein offenes Kleeblattprofil aufweisen. Der mechanische Halt in der Markhöhle, insbesondere die Verdrehsicherheit, ist bei dem Küntscher-Nagel aber ungenügend. Der Vorteil eines derartigen Nagels liegt in seiner Elastizität aufgrund eines axialen Längsschlitzes, wodurch eine gewisse Federwirkung erreicht wird und der eingeschlagene Nagel seitlich gegen das Knochenmaterial gepreßt wird.

Die beschriebenen Nägel werden zur Versorgung von Frakturen im mittleren Drittel bzw. im subtrochanteren Bereich eingesetzt. Zur Versorgung Von komplizierten Femurfrakturen sind sie allerdings nur bedingt einsetzbar.

Ein Verriegelungsnagel der eingangs genannten Art ist aus der GB-A-2 209 947 bekanntgeworden. Der bekannte Nagel weist im distalen Bereich im Querschnitt ein Kleeblattprofil auf. Ein Übergangsabschnitt zwischen distalem und proximalem Nagelabschnitt ist zylindrisch geschlossen und verbindet den proximalen Abschnitt mit einem im Durchmesser geringeren distalen Nagelabschnitt.

Aus EP-A-0 306 709 ist ein Verriegelungsnagel bekanntgeworden, der durchgehend hohl ist mit durchgehendem Kleeblattprofil, wie er als sogenannter Küntscher-Nagel bekanntgeworden ist. In den Nagel werden Innenkörper eingeschoben, die Durchbohrungen aufweisen, die mit Durchbohrungen des Nagels zur Deckung gebracht werden können. Rippenartige Abschnitte der Innenkörper wirken mit dem Längsschlitz des Nagels zusammen, um in diesem geführt zu werden.

Der Erfindung liegt somit die Aufgabe zugrunde, einen Verriegelungsnagel zu schaffen, der sicher im Femur festgelegt und mit dem komplizierte Femurfrakturen im mittleren und trochanteren Bereich versorgt werden können.

Diese Aufgabe wird gelöst durch die Merkmale des Kennzeichnungsteils des Anspruchs 1.

Der Verriegelungsnagel nach der Erfindung weist in seinem distalen Nagelabschnitt ein offenes Kleeblattprofil mit einem axialen Längsschlitz auf sowie mindestens eine Querbohrung zur Aufnahme einer Knochenschraube. Der Längsschlitz erstreckt sich im wesentlichen von dem freien distalen Nagelende bis zu einem Übergangsbereich, der den distalen Nagelabschnitt mit dem zylindrisch geschlossenen, proximalen Abschnitt verbindet. Der proximale Abschnitt weist gegebenenfalls eine Schrägdurchbohrung zur Halterung und Führung einer Schenkelhalsschraube auf.

Der kleeblattförmige distale Abschnitt hat einen geringeren Durchmesser als der zylindrische, geschlossene proximale Abschnitt, wobei der Übergangsbereich konisch ausgebildet sein kann.

Der distale Nagelabschnitt ist um 6 bis 10° in der anterior-posterior Ebene abgewinkelt zum proximalen Nagelabschnitt angeordnet. Der distale Nagelabschnitt ist ferner um 4 bis 7° in der lateral-medialen Ebene abgewinkelt zum proximalen Nagelabschnitt.

Nach einer weiteren Ausgestaltung der Erfindung ist ein Innengewinde zur Aufnahme eines Verriegelungsstiftes oberhalb der Schrägdurchbohrung angeordnet. Ein zweites Innengewinde kann am proximalen Nagelende zur Aufnahme eines Zielgerätes oder eines Einschlagwerkzeugs geformt sein.

Der erfindungsgemäße Verriegelungsnagel, dessen Länge 240 bis 480 mm beträgt, ist vorteilhafterweise zur Versorgung von komplizierten Femurfrakturen zu verwenden, wobei er einen hohen mechanischen Halt in der Markhöhle sowie eine gute Verdrehsicherheit aufweist. Durch den Längsschlitz wird eine gewisse Elastizität des Nagels erreicht. Mittels der im unteren distalen Bereich angeordneten Querbohrungen wird der Nagel durch Knochenschrauben axial und in Drehrichtung festgelegt. Der Verriegelungsnagel nach der Erfindung kann entsprechend der physiologischen Antekurvation des Femurs gebogen sein. Eine Schenkelhalsschraube kann durch die Schrägdurchbohrung geführt bzw. gehaltert und durch einen Verriegelungsstift, der in das proximale Ende des Nagels eingeführt und durch das Innengewinde festgelegt wird, in Drehrichtung gesichert werden, wobei eine axiale Bewegung aber noch zugelassen ist. Ein Einschlaginstrument, mit dem normalerweise Verriegelungsnägel eingesetzt werden, oder ein Zielgerät zur Lagebestimmung der Schrägdurchbohrung beim lateralen Anbohren des Femurs zur Einführung der Schenkelhalsschraube können in ein zweites am distalen Nagelende angeordnetes Innengewinde eingreifen.

Der erfindungsgemäße Nagel wird aus einem zylindrischen Vollrohr aus körperverträglichem, nicht-korrodierendem Material geformt. Das Rohr wird innen aufgebohrt, wobei bevorzugterweise die Innenbohrung im distalen Nagelabschnitt einen größeren Durchmesser aufweist als im proximalen Nagelabschnitt, und in einem zweiten Schritt wird der axiale Längsschlitz ausgebildet. Anschließend erfolgt eine äußere Abarbeitung des Rohres zur Herausbildung des einen im Vergleich zum proximalen Abschnitt einen geringeren Durchmesser aufweisenden, kleeblattförmigen distalen Abschnitts, des konischen Übergangsabschnitts und des zylindrischen proximalen Abschnitts. Die Querbohrungen und Innengewinde werden in bekannter Weise geformt. Das Kleeblattprofil wird durch Preßformung hergestellt.

Ein Ausführungsbeispiel der Erfindung wird nachfolgend anhand von Zeichnungen näher erläutert.
- Fig. 1: zeigt eine Seitenansicht eines Verriegelungsnagels nach der Erfindung zur Versorgung von Frakturen im rechten Femur.
- Fig. 2: zeigt einen Schnitt durch den Nagel nach Fig. 1 entlang der Linie A-A.
- Fig. 3: zeigt eine Draufsicht auf den Nagel nach Fig. 1 entlang des Pfeils B.
- Fig. 4: zeigt eine Vorderansicht, teilweise im Schnitt, des Nagels nach Fig. 1.
- Fig. 5: ist eine Dreh- und Bohrzeichnung des Verriegelungsnagels.

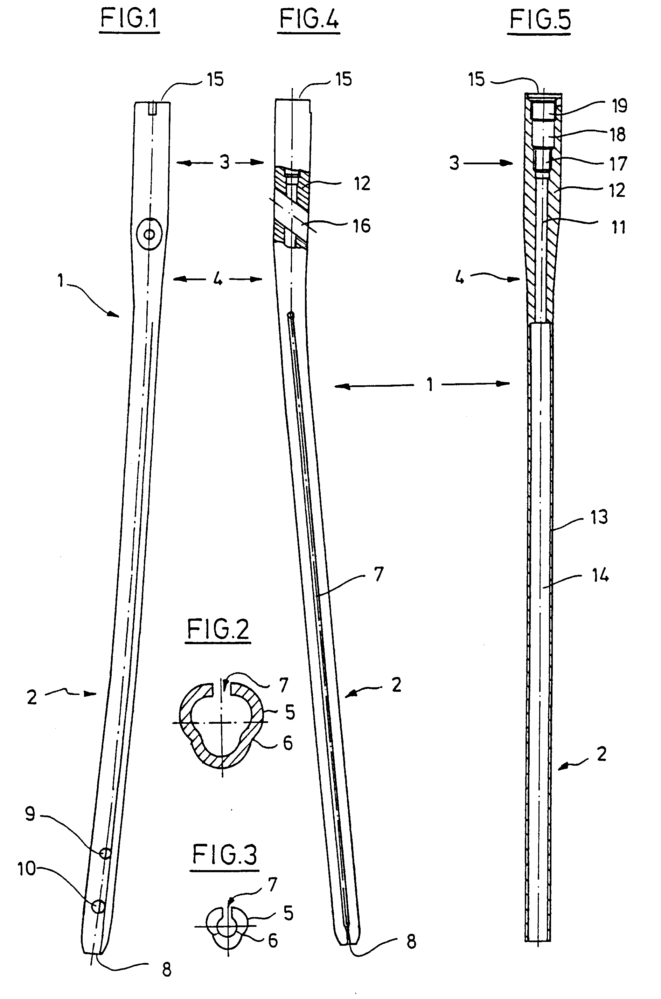

Ein Verriegelungsnagel 1 weist einen distalen Abschnitt 2, einen proximalen Abschnitt 3 und einen konisch ausgeführten Übergangsabschnitt 4 auf. Der distale Abschnitt 2 entspricht im Querschnitt (Fig. 2, 3) einem dreiblättrigen, offenen Kleeblatt mit Erhebungen 5 und Vertiefungen 6. Ein axialer Längsschlitz 7 erstreckt sich vom freien distalen Nagelende 8 über die gesamte Länge des distalen Abschnitts 2, wodurch das offene Kleeblattprofil gebildet wird. Im unteren Bereich des distalen Abschnitts 2 sind zwei Querbohrungen 9, 10 angeordnet.

Der proximale Abschnitt 3 ist im Querschnitt kreisförmig und hat einen größeren Durchmesser als der des distalen Abschnitts 2. Der Verriegelungsnagel 1 hat in seinem proximalen Abschnitt 3 eine im Verhältnis zu seiner Innenbohrung 11 große Wanddicke 12, während die distale Wanddicke 13 im Vergleich zur Innenbohrung 14 des distalen Abschnitts eher klein ist. Die Innenbohrung 11 erstreckt sich vom Ende des distalen Abschnitts bis zum freien proximalen Nagelende 15.

Eine Schrägdurchbohrung 16 für die Führung und Halterung einer Schenkelhalsschraube (nicht gezeigt) ist oberhalb des Übergangsbereichs 4 im proximalen Abschnitt 3 ausgebildet (Fig. 4), der gemäß Fig. 5 ferner ein Innengewinde 17 zur Aufnahme eines nicht dargestellten Verriegelungsstiftes aufweist. Angrenzend an das Innengewinde 17 ist die Innenbohrung 11 bis zum proximalen Nagelende 15 zylindrisch aufgeweitet. Die zylindrische Aufweitung 18 und ein nahe am proximalen Ende 15 geformtes zweites Innengewinde 19 dienen zur Aufnahme und Positionierung eines Zielgerätes oder eines Einschlagwerkzeugs.

Durch eine entsprechende Biegung des Übergangsbereichs 4 ist der geradlinige distale Abschnitt 2 in der lateral-medial Ebene um etwa 6° (Fig. 4) und in der anterior-posterior Ebene um etwa 4° (Fig. 1) abgewinkelt zum proximalen Abschnitts 3 angeordnet.

## Patentansprüche

1. Verriegelungsnagel zur Versorgung von Femurfrakturen im mittleren und im trochanteren Bereich, wobei der in den Markraum einführbare hohle Nagel (1) in seinem proximalen Abschnitt (3) gegebenenfalls eine Schrägdurchbohrung (16) zur Halterung und Führung einer Schenkelhalsschraube aufweist sowie in seinem distalen Abschnitt (2) mindestens eine Querbohrung (9, 10) zur Aufnahme einer Knochenschraube, wobei der distale Nagelabschnitt (2) ein Kleeblattprofil aufweist, und ein Übergangsabschnitt (4) einen zylindrisch geschlossenen proximalen Nagelabschnitt mit dem einen geringeren Außendurchmesser aufweisenden, distalen Nagelabschnitt (2) verbindet, gekennzeichnet durch folgende Merkmale
- die Länge des Nagels (1) ist zwischen 240 und 480 mm;
- der ein offenes Kleeblattprofil aufweisende distale Nagelabschnitt (2) weist einen axialen Längsschlitz (7) auf, der sich vom freien distalen Nagelende (8) bis zum Übergangsabschnitt (4) erstreckt;
- in der lateral-medial Ebene des Verriegelungsnagels (1) ist der distale Nagelabschnitt (2) um 6 bis 10° abgewinkelt zum proximalen Nagelabschnitt (3) angeordnet;
- in der anterior-posterior Ebene des Verriegelungsnagels (1) ist der distale Nagelabschnitt (2) um 4 bis 7° abgewinkelt zum proximalen Nagelabschnitt (3) angeordnet und
- die Wanddicke des Übergangs- und des proximalen Nagelabschnitts (3, 4) ist groß im Verhältnis zu der Wanddicke im Bereich des Kleeblattprofils (2).

2. Verriegelungsnagel nach einem der Ansprüche 1 bis 3, dadurch gekennzeichnet, daß der Übergangsabschnitt (4) konisch von dem proximalen (3) in den distalen Abschnitt (2) übergeht.

3. Verriegelungsnagel nach Anspruch 1 oder 2, dadurch gekennzeichnet, daß im proximalen Nagelabschnitt (3) ein Innengewinde (17) zur Aufnahme eines Verriegelungsstifts geformt ist.

4. Verriegelungsnagel nach Anspruch 1 und 3, dadurch gekennzeichnet, daß nahe am proximalen Nagelende (15) ein zweites Innengewinde (19) zur Aufnahme eines Zielgerätes oder eines Einschlagwerkzeugs geformt ist.

## Claims

1. A locking nail for the treating of femoral fractures in the medial and trochanter region, with the proximal portion (3) of the hollow nail (1) adapted to be introduced in the medullary canal occasionally including an oblique throughbore (16) for retaining and guiding a femur neck screw, and with its distal portion (2) including at least one transverse throughbore (9, 10) for the receipt of a bone screw, the distal nail portion (2) having a clover-leaf profile, and a transition portion (4) interconnecting a cylindrically closed proximal nail portion with the distal nail portion (2) having a smaller outer diameter, characterized by the following features:
- the length of the nail (1) is between 240 and 480 mm;
- the distal nail portion (2) having an opened clover-leaf profile has an axial longitudinal slot (7) extending from the free distal end of the nail up to the transition portion (4);
- in the lateral medial plane of the nail (1) the distal nail portion (2) is angled about 6 to 10° relative to the proximal portion (3);
- in the anterior-posterior plane the distal nail portion (2) is angled about 4 to 7° relative to the proximal nail portion, and
- the thickness of the wall of the transition and the proximal nail portion (3, 4) is large if compared with the thickness of the wall in the region of the clover-leaf profile.

2. The locking nail of claim 1 or 2, wherein the transition portion (4) extends conically from the proximal (3) to the distal portion (2).

3. The locking nail of claim 1 or 2, wherein the proximal nail portion (3) has an internal thread (17) for the receipt of a locking pin.

4. The locking nail of claims 1 and 3, wherein a second internal thread (19) is formed adjacent to the proximal end of the nail for the receipt of a targeting device or a tool.

## Revendications

1. Broche d'immobilisation pour soigner des fractures du fémur dans la région médiane et du trochanter, où la broche creuse (1) pouvant être introduite dans l'espace médullaire présente dans sa section proximale (3), le cas échéant, un passage oblique (16) pour la fixation et le guidage d'une vis de col du fémur, ainsi qu'au moins un perçage transversal (9, 10) pour la réception d'une vis à os, dans sa section distale (2), cette section distale (2) présentant un profil en feuille de trèfle et une section de transition (4) reliant une section proximale de broche cylindrique close avec la section distale de broche (2) présentant un diamètre extérieur inférieur, caractérisée par les particularités suivantes :
- la longueur de la broche (1) est comprise encre 240 et 480 mm ;
- la section distale (2) de broche ayant un profil en feuille de trèfle fendue présente une fente oblongue axiale (7) qui s'étend de l'extrémité distale libre (8) de la broche jusqu'à la section de transition (4) ;
- la section distale (2) de broche est disposée recourbée d'un angle de 6 à 10° avec la section proximale (3) de broche dans le plan latéral-médial de la broche d'immobilisation (1) ;
- la section distale (2) de broche est disposée recourbée d'un angle de 4 à 7° avec la section proximale (3) de broche dans le plan antérieur-postérieur de la broche d'immobilisation (1) et
- l'épaisseur de paroi des sections de transition et proximale (3, 4) est importante par rapport à l'épaisseur de paroi dans la zone du profil en feuille de trèfle (2).

2. Broche d'immobilisation selon la revendication 1, caractérisée en ce que la section de transition (4) passe de manière conique de la section proximale (3) à la section distale (2).

3. Broche d'immobilisation selon la revendication 1 ou 2, caractérisée en ce qu'un taraudage (17) de réception d'une tige de blocage est formé dans la section proximale (3) de broche.

4. Broche d'immobilisation selon les revendications 1 et 3, caractérisée en ce qu'un deuxième taraudage (19) de réception d'un appareil de visée ou d'un outil d'enfoncement est formé près de l'extrémité proximale (15) de la broche.
